# EUROPÄISCHE PATENTSCHRIFT

(11) **EP 1 331 903 B1**
(45) Veröffentlichungstag und Bekanntmachung des Hinweises auf die Patenterteilung: **23.06.2004**
(21) Anmeldenummer: 01993438.9
(22) Anmeldetag: 12.10.2001
(51) Int. Cl.: A61F 2/02, A61B 17/72

(54) **EINRICHTUNG ZUR STEUERUNG, REGELUNG UND/ODER ZUR INBETRIEBNAHME EINES AKTIVEN IMPLANTATES**
DEVICE FOR CONTROLLING, REGULATING AND/OR PUTTING AN ACTIVE IMPLANT INTO OPERATION
DISPOSITIF POUR COMMANDER, REGULER ET/OU METTRE EN FONCTIONNEMENT UN IMPLANT ACTIF

(30) Priorität: 09.11.2000 DE 10055519
(43) Veröffentlichungstag der Anmeldung: 06.08.2003
(73) Patentinhaber: Wittenstein AG, 97997 Igersheim (DE)
(72) Erfinder: STAUCH, Roman, 97959 Assamstadt (DE); WITTENSTEIN, Manfred, 97980 Bad Mergentheim (DE)
(74) Vertreter: Weiss, Peter, Dr.
(86) Internationale Anmeldenummer: PCT/EP2001/011809
(87) Internationale Veröffentlichungsnummer: WO 2002/038082

(56) Entgegenhaltungen:
- EP-A- 0 820 731
- WO-A-00/33751
- WO-A-96/25117
- DE-A- 19 700 225
- DE-A- 19 856 013
- FR-A- 2 726 460
- US-A- 5 626 579

## Beschreibung

Die vorliegende Erfindung betrifft eine Einrichtung zur Steuerung, Regelung und/oder zur Inbetriebnahme eines aktiven Implantates, insbesondere Distraktionsvorrichtung, welche mit einer Empfängereinheit implantierbar und über eine Sendereinheit Daten und/oder Energie von aussen zuführbar ist.

Derartige Einrichtungen zur Steuerung und/oder zur Inbetriebnahme eines aktiven Implantates, insbesondere von Distraktionsvorrichtungen, sind in vielfältiger Form und Ausführung auf dem Markt bekannt und gebräuchlich. Sie dienen im wesentlichen zur Distraktion von beliebigen Knochen, Knochenteilen, Knochensegmenten aber auch zum Einsetzen in Markräume von Knochen.

Es ist im Stand der Technik bekannt, dass beispielsweise an ein Implantat ein Stecker anschliesst, der zu einer subkutan implantierbaren Empfängereinheit führt, die dann von aussen berührungslos mittels einer Sendereinheit beispielsweise mechanisch aktivierbar ist.

Nachteilig hieran ist, dass den Patienten die subkutan implantierte Empfängereinheit, bspw. wenn diese einen längeren Zeitraum implantiert sein muss, stört und gegebenenfalls Schmerzen bereitet.

Ferner ist eine derartige mit einem Kabel verbundene Empfängereinheit anfällig gegen Beschädigungen, wenn diese bspw. operativ eingesetzt wird. Eine derartige Empfängereinheit ist nur schwer austauschbar, sollte diese beschädigt sein. Meist muss das vollständige Implantat entfernt werden oder ausgetauscht werden, was zu unerwünschten Kosten und Operationszeiten sowie Risiken einer erneuten Operation für den Patienten führt.

Die DE 197 00 225 A1 (Basis für den Oberbegriff des Anspruchs 1) offenbart eine Distraktionsvorrichtung zum Auseinanderbewegen zweier Teile eines Knochens. Dabei ist ein Marknagel zweiteilig ausgebildet, wobei in einem Teil des Marknagels eine Arbeitseinrichtung vorgesehen ist, die über eine elektrische Leitung von einer Induktionsspule versorgbar ist. Die Induktionsspule ist subkutan implantierbar.

Die DE 198 56 013 A1 beschreibt eine Distraktionsvorrichtung zur operativen Korrektur von Wirbelsäulenerkrankungen sowie Schädigungen oder Missbildungen. Diese besteht aus zwei gegeneinander bewegbaren Elementen, die berührungslos über ein Energieübertragungselement Energie an die Distraktionsvorrichtung überträgt. Dabei ist das Energieübertragungselement mittels einer Verbindungsleitung an die Distraktionsvorrichtung angeschlossen. Eine ähnliche Distraktionsvorrichtung ist in der Internationalen Patentanmeldung WO00/33751 beschrieben. Dort wird auch über ein Energieübertragungselement, welches über eine Verbindungsleitung an einer Betätigungseinrichtung anschliesst, Energie übertragen.

Der vorliegenden Erfindung liegt die Aufgabe zugrunde, eine Einrichtung der eingangs genannten Art zu schaffen, welche die genannten Nachteile beseitigt, und mit welcher sehr kostengünstig und effektiv eine Empfängereinheit ausgetauscht werden kann und eine Energie- und/oder Datenübertragung erheblich verbessert werden soll. Zudem sollen Operationszeiten verkürzt werden.

Zur Lösung dieser Aufgabe führt, dass die Empfängereinheit stirnseitig wiederlösbar mit einem Endstück des Implantates zum Herstellen der mechanischen und elektrischen Verbindung als Schnittstelle von der Empfängereinheit zum Implantat verbindbar ist.

Bei der vorliegenden Erfindung schliesst vorzugsweise die Empfängereinheit an ein Endstück des Implantates wieder lösbar an. Dies erfolgt vorzugsweise mechanisch, indem beispielsweise ein Gewindezapfen oder Gewindestift der Empfängereinheit in ein passendes Innengewinde stirnseitig in das Implantat eingreift und somit eine Zentrierung und mechanische Verbindung herstellt. Diese sind wie auch andere Verbindungsmöglichkeiten mechanischer Art denkbar.

Bspw. können Steckrastverbindungen vorgesehen sein, um wieder lösbar die Empfängereinheit dem Implantat vorzugweise stirnseitig aufzusetzen.

Wichtig ist jedoch, dass durch das Verbinden der Empfängereinheit mit dem Implantat, insbesondere mit dem Endstück des Implantates gleichzeitig eine Verbindung zur Übertragung von induktiven und/oder elektrischen Signalen zwischen Empfängereinheit und Implantat hergestellt wird. Hierzu können entsprechende Kontaktierungen auf einem Absatz der Empfängereinheit vorgesehen sein, die dann in entsprechende passende Kontaktstellen, Kontaktflächen oder dergleichen des Implantates passen. Die Energie- sowie Datenübertragung kann auch induktiv zwischen Empfängereinheit und Implantat erfolgen. In diesem Fall entfallen mechanische Kontakte.

Hierdurch ist gewährleistet, dass über eine entsprechende externe Sendereinheit, die lediglich auf die Haut aufgelegt wird, bspw. eine induktive Energieübertragung und eine Datenübertragung zum aktiven Implantat, bidirektional, was die Datenübertragung betrifft, erfolgt.

Vorzugsweise wird das Implantat in einen Markraum des Knochens implantiert und anschliessend nach dem Einbringen des Implantates wird nach dem Verriegeln des Implantates gegenüber dem Knochen, der beispielsweise verlängert werden soll, die Empfängereinheit in das Implantat eingeschraubt und stellt eine mechanische sowie auch eine elektrische Schnittstelle zum Implantat einerseits und andererseits zur Sendereinheit her.

Dabei kann die Sendereinheit in jeder beliebigen Position bspw. rechtwinklig seitlich die Energie auf die Empfängereinheit übertragen. Allerdings ist auch eine stirnseitige Übertragung von Energie und/oder Daten von Sendereinheit auf die Empfängereinheit möglich. Hierdurch lassen sich viele mögliche Anwendungsfälle insbesondere zur Knochendistraktion realisieren, so dass sehr angenehm, ohne dass eine subkutan störende separat eingesetzte Empfängereinheit besteht, Energie und/oder Daten ausgetauscht werden können. Dabei kann die Sendereinheit von mehreren beliebigen Seiten auf die Haut von aussen aufgelegt werden. Auch kann daran gedacht sein, bspw. die Sendereinheit als anlegbare Manschette, als Ringspule od. dgl. ringförmig auszugestalten, um diese bspw. über einen Arm oder ein Bein stülpen oder zu legen, um die Empfängereinheit mit Energie und/oder Daten zu versorgen. Dies soll ebenfalls im Rahmen der vorliegenden Erfindung liegen.

Weitere Vorteile, Merkmale und Einzelheiten der Erfindung ergeben sich aus der nachfolgenden Beschreibung bevorzugter Ausführungsbeispiele sowie anhand der Zeichung; diese zeigt in
Figur 1 eine schematisch dargestellte Draufsicht auf eine Einrichtung zur Steuerung, Regelung und/oder zur Inbetriebnahme eines aktiven Implantates;
Figur 2 eine schematisch dargestellte Draufsicht auf die Einrichtung gemäss Figur 1 in einer anderen Gebrauchslage.

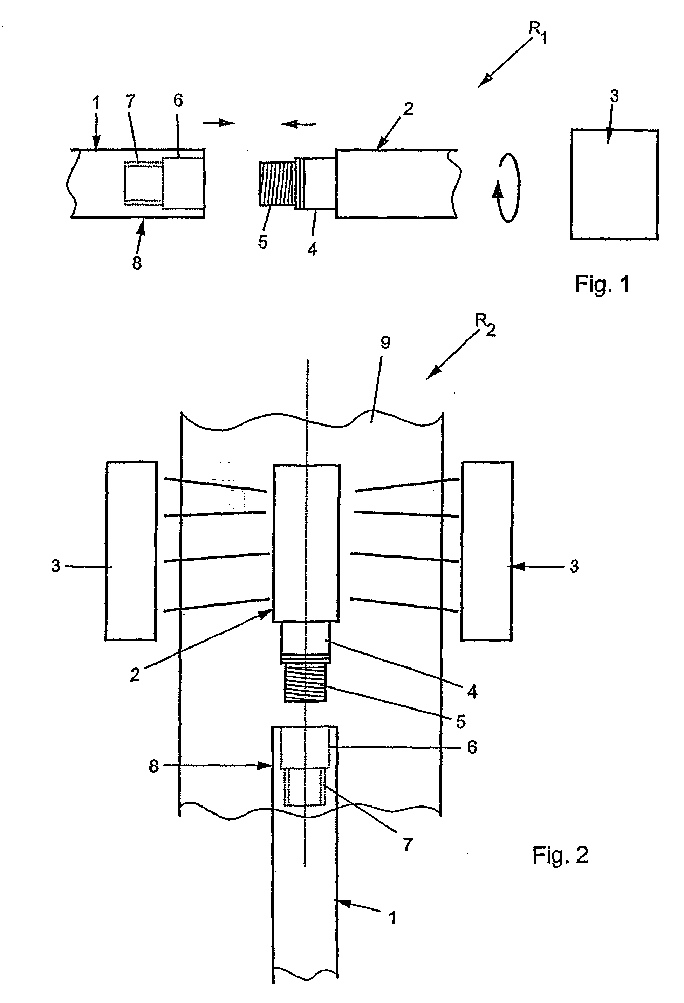

Gemäss Figur 1 weist eine erfindungsgemässe Einrichtung R₁ zur Steuerung, Regelung und/oder zur Inbetriebnahme eines aktiven Implantates 1 eine Empfängereinheit 2 auf, welche über eine Sendereinheit 3 berührungslos betätigbar ist. Im bevorzugten Ausführungsbeispiel ist die Empfängereinheit 2 querschnittlich rund und länglich ausgebildet und ist endseits mit einem Absatz 4 versehen, der sich stirnseitig der Empfängereinheit 2 anschliesst. Dabei ist der Absatz 4 vorzugsweise auch querschnittlich rund ausgebildet und beinhaltet entsprechende Kontaktspuren, Spulenelemente oder dergleichen, um Daten und/oder Energie von der Empfängereinheit 2 auf das Implantat 1 zu übertragen. Im Anschluss an den Absatz 4 schliesst ein Gewinde 5, welches als Aussengewinde ausgebildet ist, an. Vorzugsweise ist das Gewinde 5 mit einer hier nicht näher dargestellten Zentrierung versehen, um insbesondere den Absatz 4 passgenau in eine entsprechende Kontaktfläche 6 des Implantates 1 zu zentrieren und insbesondere zu positionieren. Die Energie- und/oder Datenübertragung kann anstelle der mechanischen Kontakte auch induktiv und berührungslos, auch bidirektional erfolgen.

Es hat sich bei der vorliegenden Erfindung als besonders vorteilhaft erwiesen, insbesondere ein Endstück 8 des Implantates 1 mit einer inneren Kontaktfläche 6 und anschliesendem Innengewinde 7 zu versehen, um die Empfängereinheit 2 stirnseitig mit dem Implantat 1 zu verbinden.

Bei der vorliegenden Erfindung wird das Implantat 1 bspw. stirnseitig zur Distraktion von Knochen in einen Markraum der jeweiligen Gliedmassen 9 eingeführt. Nach dem Einsetzen und Verankern des Implantates 1 in den Markraum 9 wird anschliessend die Empfängereinheit 2 stirnseitig auf das Endstück 8 des Implantates 1 aufgeschraubt, wodurch gleichzeitig die elektrischen Verbindungen und Kontakte zur Daten- und/oder Energieübertragung zwischen Empfängereinheit 2 und Implantat 1 hergestellt werden. Während der Implantationen bzw. Operationen ist die Schnittstelle der Kontaktfläche 6 bzw. das Innengewinde 7 vor Beschädigungen durch Instrumentarien geschützt.

Dies hat zum Vorteil, dass die empfindliche Empfängereinheit 2 während der Operation, insbesondere während des Einsetzens des Implantates 1 in den Markraum 9 eines Knochens, nicht beschädigt oder zerstört wird.

Ferner ist von Vorteil, dass insbesondere durch das stirnseitige Anschliessen der Empfängereinheit 2 an das Implantat 1 diese mit dem Implantat 1 vollständig in den zu verlängernden Knochen eingesetzt wird, ohne dass eine beispielsweise flexible Kabelverbindung zwischen Implantat und Empfängereinheit 2 hergestellt werden müssen, die subkutan unangenehm unter der Haut liegen und nur bedingt haltbar ist.

Auch ist von Vorteil, dass die Empfängereinheit 2 jederzeit wieder austauschbar ist, sollte diese bspw. durch eine andere ersetzt werden, die eine andere Leistung oder einen anderen Datenaustausch gewährleisten. Dies soll ebenfalls im Rahmen der vorliegenden Erfindung liegen.

Vorzugsweise ist die Empfängereinheit 2 querschnittlich rund und länglich ausgebildet, sie kann jedoch auch andere Formen annehmen.

In Figur 2 ist eine die Einrichtung R₂ dargestellt, bei welcher die Sendereinheit 3 seitlich oder, wie in Figur 1 dargestellt, stirnseitig ausserhalb der Haut bzw. auf die Haut aufgelegt werden kann, um die Empfängereinheit 2 entsprechend mit Daten und/oder Energie zu versorgen. Diese kann bspw. flexibel oder starr als Manschette oder als Ring ein- oder mehrteilig, auch unterteilbar, ausgebildet sein. Hierdurch lassen sich sehr viele Einsatzgebiete erschliessen, wenn insbesondere die Empfängereinheit 2 seitlich durch die Sendereinheit 3 oder stirnseitig, wie in Figur 1 dargestellt ist, betätigbar ist.

Ferner soll daran gedacht sein, radial und ggf. koaxial eine Sendereinheit 3 ringförmig auszubilden, um die Empfängerbereiche 2 vollständig zu umfangen. Es können auch mehrere einzelne Sendereinheiten 3 radial aussen und/oder strinseitig auf die Haut aufgelegt werden, um Energie und/oder Daten zu übertragen.

| **Positionszahlenliste** | | | | | |
|---|---|---|---|---|---|
| 1 | Implantat | 34 | | 67 | |
| 2 | Empfängereinheit | 35 | | 68 | |
| 3 | Sendereinheit | 36 | | 69 | |
| 4 | Absatz | 37 | | 70 | |
| 5 | Gewinde | 38 | | 71 | |
| 6 | Kontaktfläche | 39 | | 72 | |
| 7 | Innengewinde | 40 | | 73 | |
| 8 | Endstück | 41 | | 74 | |
| 9 | Gliedmassen/ Markraum | 42 | | 75 | |
| 10 | | 43 | | 76 | |
| 11 | | 44 | | 77 | |
| 12 | | 45 | | 78 | |
| 13 | | 46 | | 79 | |
| 14 | | 47 | | | |
| 15 | | 48 | | | |
| 16 | | 49 | | R₁ | Einrichtung |
| 17 | | 50 | | R₂ | Einrichtung |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. , Einrichtung zur Steuerung, Regelung und/oder zur Inbetriebnahme eines aktiven Implantates (1), insbesondere einer Distraktionsvorrichtung, welche mit einer Empfängereinheit (2) implantierbar ist und zu welcher über eine Sendereinheit (3) Daten und/oder Energie von aussen zuführbar sind,
**dadurch gekennzeichnet,**
**dass** die Empfängereinheit (2) stirnseitig wiederlösbar mit einem Endstück (8) des Implantates (1) zum Herstellen der mechanischen und elektrischen Verbindung als Schnittstelle von der Empfängereinheit (2) zum Implantat (1) verbindbar ist.

2. Einrichtung nach Anspruch 1, **dadurch gekennzeichnet, dass** die Empfängereinheit (2) nach dem Einsetzen des Implantates (1) in einen Markraum eines Gliedmasses (9) eines Knochens, zum Herstellen von elektrischen Kontakten oder Verbindungen für eine Energieübertragung und/oder Datenaustausch, ggf. bidirektional, verbindbar ist.

3. Einrichtung nach Anspruch 1 oder 2, **dadurch gekennzeichnet, dass** die Empfängereinheit (2) wenigstens einen Absatz (4) zur internen Kontaktierung und Übertragung von Daten und/oder Energie an das Implantat (1) aufweist, an welchen sich ein Gewinde (5) ggf. mit Zentrierung anschliesst, oder ggf. Bajonettverschlüsse, Steck- Rastverbindungen od. dgl. vorgesehen sind.

4. Einrichtung nach wenigstens einem der Ansprüche 1 bis 3, **dadurch gekennzeichnet, dass** stirnseitig das Implantat (1) mit einem entsprechenden Innengewinde (7) und einer entsprechenden Kontaktfläche (6) für Kontakte zumindest einen Absatzes (4) der Empfängereinheit (2) vorgesehen ist.

5. Einrichtung nach wenigstens einem der Ansprüche 1 bis 4, **dadurch gekennzeichnet, dass** die Empfängereinheit (2) querschnittlich rund und länglich ausgebildet ist,

6. Einrichtung nach wenigstens einem der Ansprüche 1 bis 5, **dadurch gekennzeichnet, dass** die Empfängereinheit (2) in das Endstück (8) zum Herstellen der mechanischen und elektrischen Verbindung als Schnittstelle von der Empfängereinheit (2) zum Implantat (1) einschraubbar ist.

7. Einrichtung nach wenigstens einem der Ansprüche 1 bis 6, **dadurch gekennzeichnet, dass** die Empfängereinheit (2) jederzeit austauschbar und ersetzbar mit dem Implantat (1) verbindbar ist.

8. Einrichtung nach wenigstens einem der Ansprüche 1 bis 7, **dadurch gekennzeichnet, dass** über die Sendereinheit (3) von aussen, insbesondere über induktive Energieübertragung Energie der Empfängereinheit (2) zugeleitet wird, welche die induktiv eingekoppelte Energie dem Implantat (1) zum in Betrieb setzen einer Antriebseinrichtung, insbesondere Micromotor zuführt.

9. Einrichtung nach wenigstens einem der Ansprüche 1 bis 8, **dadurch gekennzeichnet, dass** die Empfängereinheit (2) Daten, wie telemetrische Daten von Sensorsignalen, Wegsignalen, Kraftsignalen der Sendereinheit (3), auch bidirektional übermittelt.

10. Einrichtung nach wenigstens einem der Ansprüche 1 bis 9, **dadurch gekennzeichnet, dass** die Sendereinheit (3) manschettenartig, kreisringartig ausgebildet ist, und ggf. koaxial die Empfängereinheit (2) zum Austausch von Energie und/oder Daten übergreift.

11. Einrichtung nach Anspruch 10, **dadurch gekennzeichnet, dass** die Sendeeinheit (3) flexibel oder starr ausgebildet und koaxial, seitlich oder stirnseitig den Gliedmassen (9) von aussen auflegbar ist.

12. Einrichtung nach wenigstens einem der Ansprüche 1 bis 11, **dadurch gekennzeichnet, dass** zwischen Empfangereinheit (2) und Implantat (1) eine Ernergieübertragung und/oder ein Datenaustausch, qqf. auch bidirektional, induktiv erfolgt.

## Claims

1. Means for controlling, regulating and/or putting into operation an active implant (1), more especially a distraction device, which can be implanted with a receiver unit (2), and to which data and/or energy can be supplied from externally via a transmitter unit (3), **characterised in that** the receiver unit (2) is re-releasably connectable, at its end face, to an end-piece (8) of the implant (1) in order to establish the mechanical and electrical connection in the form of an interface between the receiver unit (2) and the implant (1).

2. Means according to claim 1, **characterised in that**, after the insertion of the implant (1) into a marrow cavity of a limb (9) of a bone, the receiver unit (2) is connectable in order to establish electrical contacts or connections for a transfer of energy and/or data exchange, possibly bi-directionally.

3. Means according to claim 1 or 2, **characterised in that** the receiver unit (2) has at least one shoulder portion (4) for internal contacting purposes and for the transfer of data and/or energy to the implant (1), with which shoulder portion a thread (5), possibly with a centring means, communicates, or possibly bayonet-type locking members, plug-in connections, locking connections or the like are provided.

4. Means according to least one of claims 1 to 3, **characterised in that** the implant (1) is provided, on its end face, with a corresponding internal thread (7) and a corresponding contact face (6) for contact with at least one shoulder portion (4) of the receiver unit (2).

5. Means according to at least one of claims 1 to 4, **characterised in that** the receiver unit (2) has a circular cross-section and is elongate.

6. Means according to least one of claims 1 to 5, **characterised in that** the receiver unit (2) is screw-connectable into the end-piece (8) in order to establish the mechanical and electrical connection in the form of an interface between the receiver unit (2) and the implant (1).

7. Means according to at least one of claims 1 to 6, **characterised in that** the receiver unit (2) is connectable so as to be exchangeable and replaceable with the implant (1) at any time.

8. Means according to at least one of claims 1 to 7, **characterised in that** energy is supplied to the receiver unit (2) via the transmitter unit (3) from externally, more especially via an inductive transfer of energy, which receiver unit supplies the inductively coupled-in energy to the implant (1) in order to put into operation a driving means, more especially a micro-motor.

9. Means according to at least one of claims 1 to 8, **characterised in that** the receiver unit (2) transfers data, such as telemetric data of sensor signals, directional signals and power signals of the transmitter unit (3), even bi-directionally.

10. Means according to at least one of claims 1 to 9, **characterised in that** the transmitter unit (3) has a sleeve-like, circular ring-shaped configuration and possibly overlaps coaxially the receiver unit (2) for the exchange of energy and/or data.

11. Means according to claim 10, **characterised in that** the transmitter unit (3) has a flexible or rigid configuration and can be mounted coaxially of, laterally of or on the end face of the limbs (9) from externally.

12. Means according to at least one of claims 1 to 11, **characterised in that** a transfer of energy and/or a data exchange, possibly even bi-directionally, are/is inductively effected between the receiver unit (2) and implant (1).

## Revendications

1. Dispositif pour commander, réguler et/ou mettre en fonctionnement un implant actif (1), en particulier, un dispositif de distraction qui peut être implanté avec une unité de réception (2) vers laquelle peuvent être alimentées de l'extérieur, par l'intermédiaire d'une unité d'émission (3), des données et/ou de l'énergie,
**caractérisé par le fait**
**que** l'unité de réception (2) peut être raccordée, du côté frontal, de manière amovible à un embout (8) de l'implant (1), pour établir la connexion mécanique et électrique comme interface entre l'unité de réception (2) et l'implant (1).

2. Dispositif selon la revendication 1, **caractérisé par le fait que** l'unité de réception (2) peut être raccordée, après introduction de l'implant (1) dans une zone de moelle d'une masse de membre (9) d'un os, pour établir des contacts électriques ou des connexions pour une transmission d'énergie et/ou un échange de données, éventuellement de manière bidirectionnelle.

3. Dispositif selon la revendication 1 ou 2, **caractérisé par le fait que** l'unité de réception (2) présente au moins un épaulement (4) pour la mise en contact interne et la transmission de données et/ou d'énergie à l'implant (1), auquel se raccorde un filet (5), éventuellement avec centrage, ou qu'il est éventuellement prévu une connexion à baionnette, des connexions enfichables ou par encliquetage ou autres.

4. Dispositif selon au moins l'une des revendications 1 à 3, **caractérisé par le fait que** l'implant (1) est pourvu, du côté frontal, d'un filet intérieur (7) approprié et d'une surface de contact (6) appropriée pour les contacts d'au moins un épaulement (4) de l'unité de réception (2).

5. Dispositif selon au moins l'une des revendications 1 à 4, **caractérisé par le fait que** l'unité de réception (2) est réalisée de section ronde et allongée.

6. Dispositif selon au moins l'une des revendications 1 à 5, **caractérisé par le fait que** l'unité de réception (2) peut être vissée dans l'embout (8) pour établir la connexion mécanique et électrique comme interface entre l'unité de réception (2) et l'implant (1).

7. Dispositif selon au moins l'une des revendications 1 à 6, **caractérisé par le fait que** l'unité de réception (2) peut être reliée à l'implant (1) de manière échangeable et remplaçable à tout moment.

8. Dispositif selon au moins l'une des revendications 1 à 7, **caractérisé par le fait que** par l'intermédiaire de l'unité d'émission (3) est alimentée de l'extérieur, en particulier par transmission inductive, de l'énergie à l'unité de réception (2) qui alimente l'énergie couplée inductivement vers l'implant (1), pour la mise en fonctionnement d'un dispositif d'entraînement, en particulier un micromoteur.

9. Dispositif selon au moins l'une des revendications 1 à 8, **caractérisé par le fait que** l'unité de réception (2) transmet des données, telles que des données télémétriques de signaux de capteur, des signaux de trajet, des signaux de force, de l'unité d'émission (3), également de manière bidirectionnelle.

10. Dispositif selon au moins l'une des revendications 1 à 9, **caractérisé par le fait que** l'unité d'émission (3) est réalisée en forme de manchette, de bague circulaire, et vient éventuellement en prise, de manière coaxiale, par dessus l'unité de réception (2), pour l'échange d'énergie et/ou de données.

11. Dispositif selon la revendication 10, **caractérisé par le fait que** l'unité d'émission (3) est réalisée souple ou rigide et peut être placée depuis l'extérieur, coaxialement, latéralement ou du côté frontal, sur les masses de membre (9).

12. Dispositif selon au moins l'une des revendications 1 à 11, **caractérisé par le fait qu'**entre l'unité de réception (2) et l'implant (1) a lieu inductivement un transfert d'énergie et/ou un échange de données, éventuellement aussi de manière bidirectionnelle.
